# EUROPEAN PATENT APPLICATION

(11) **EP 1 902 756 A1**
(43) Date of publication of application: **26.03.2008**
(21) Application number: 07017618.5
(22) Date of filing: 08.09.2007
(51) Int. Cl.: A61Q 19/00, A61K 8/49, A61K 8/60, A61P 17/00, A61K 31/435, A61K 31/365, A61K 31/357, A61K 31/7028

(54) **Composition and method for treating skin by administering silybin and piperine/terahydropiperine**

(30) Priority: 19.09.2006 EP 06019577
(71) Applicant: Cognis IP Management GmbH, 40589 Düsseldorf (DE)
(72) Inventor: Danoux, Louis, 54420 Saulxures les Nancy (FR); Moussou, Philippe, 54510 Tomblaine (FR); Moser, Philippe, 54270 Essey Les Nancy (FR); Bailly, Laurent, 54270 Essey Les Nancy (FR); Markert, Thomas, 40789 Monheim (DE)

(57) **Abstract**

Suggested are cosmetic compositions comprising in a cosmetically acceptable excipient (a) silybin and (b) piperine and/or tetrahydropiperine. The compositions are suitable for preventing and/or soothing dry skin or reactive skin against external stresses.

## Description

### Field of the invention

The present invention is related to the area of cosmetics and refers to compositions for fighting skin harmed by stress factors, the use of certain agents for making or in said compositions.

### Background of the invention

One of the main functions of the skin, the largest bodily organ is to protect the organism against external strains such as UV radiation, pollutants, temperature stress and the like. Stress induced skin reactions may also cause by themselves additional toxic effects, thus worsening the consequences of the initial stressor. In particular, several kinds of metallic compounds such as nickel, chromate in cement and stainless steel with nickel, gold and mercury are able to cause a skin discomfort. Among them, nickel is the most mentioned and is known to induce adverse skin reactions such as skin redness, itching.

Various literature papers address the cosmetic treatment of dry and reactive skin. In this context reference made to the survey of Chavigny [Parfums Cosmetiques Actualites, 2004, 178, 128-169] reviewing the different strategies and products to increase the skin tolerance threshold, via restoration of the cutaneous barrier and via limiting the production of the cellular mediators involved in the inflammatory reaction. However there is an increasing demand to provide alternative or better agents that are suitable for preventing and/or treating dry or reactive skin.

The problem underlying the present invention has therefore been to develop a new cosmetic composition which can be applied oral and/or topical to overcome the disadvantages of the prior art and in particular to offer compositions comprising agents to prevent or to sooth dry skin or reactive skin against external stresses inducing adverse skin reactions as explained above.

### Detailed description of the invention

The present invention refers to cosmetic compositions comprising in a cosmetically acceptable excipient
(a) silybin and
(b) piperine and/or tetrahydropiperine

In a further embodiment of the invention the compositions further comprises at least one agent selected from the group consisting of nortrachelogenine, rhapontin, arctiin, artigenin and mixtures thereof.

The substances silybin, piperine, tetrahydropiperine, nortrachelogenine, rhapontin, arctiin, artigenin are herein referred to as "agents" or "active principles".

The agents are usually present in amounts of 0.0001 to 5, and preferably 0.001 to 1 % b.w. - calculated on the final composition. Suitable excipients are for example cosmetic oils or simply water.

### Piperine and/or Tetrahydropiperine

Piperine is an alkaloid found naturally in plants belonging to the *Piperaceae* family, such as *Piper nigrum* L, commonly known as black pepper, and *Piper longum* L, commonly known as long pepper. Piperine is the major pungent substance in these plants and is isolated from the fruit of the black pepper and long pepper plants. Piperine is a solid substance. Its molecular formula is C₁₇H₁₉NO₃, and its molecular weight is 285.34 daltons. Piperine is the trans-trans stereoisomer of 1-piperoylpiperidine. It is also known as (E, E)-1-piperoylpiperidine and (E, E)-1-[5-(1, 3-benzodioxol-5-yl)-1-oxo-2, 4-pentadienyl] piperidine. It is represented by the following chemical structure:

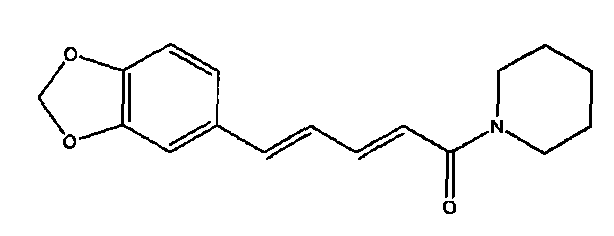

Piperine may have bioavailability-enhancing activity for some nutritional substances and for some drugs. It has putative anti-inflammatory activity and may have activity in promoting digestive processes. Piperine has been reported to increase the serum levels and lengthen the serum half lives of some nutritional substances, such as coenzyme Q₁₀ and beta-carotene, and of some drugs, such as propanolol and theophylline. Piperine, an alkaloid derived from black pepper increases serum response of beta-carotene during 14-days of oral beta-carotene supplementation. A suitable derivative of piperin is tetrahydropiperine, which can be obtained e.g. by chemical hydrogenation of piperine. Piperine or tetrahyropiperine can be used at doses between 0.0001% and 5%, preferentially at doses between 0.005% and 0.5% and more preferentially at doses between 0.02% and 0.2%. Preferably Piperine is used.

### Silybin

Seeds of *Silybum marianum* (L.) Gaertn. (*Carduus marianus* L., Asteraceae) (milk thistle) have been used for centuries to treat liver disorders or dysfuntions (cirrhosis, hepatitis), and to protect the liver against poisoning from chemical and environmental toxins (alcohol, mushroom poisoning , snake bites, insect stings).The active components of the *S. marianum* is a standardized extract obtained from the seeds and is known as silymarin. Traditionally silymarin is a mixture containing 70 % to 80 % of flavonolignans mixture and approximately 20 % to 30 % chemically undefined fraction, comprising polyphenolic compounds. The main component of the silymarin complex is silybin (CAS No. 22888-70-6), synonym with silibinin, sometimes incorrectly called silybinin according to the following formula:

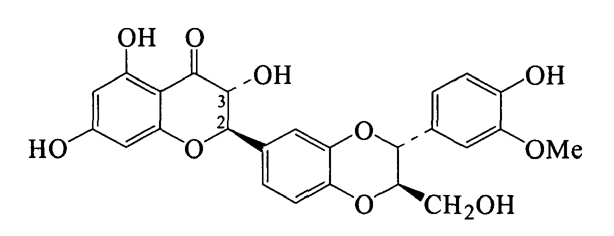

Silybin is a mixture of two diastereoisomers, silybin A and silybin B that are present approximately at 1 : 1 ratio. The term "silybin" as used throughout the invention, encompasses silybin A alone, silybin B alone as well as any mixture thereof, preferably the 1 :1 mixture. Besides silybin, other flavonolignans are present in the silymarin mixture, namely isosilybin, dehydrosilybin, silychristin, silydianin, and a few flavonoids, mainly taxifolin. Typical applications, for which milk thistle preparations have been used are mostly treatments of liver diseases. Also currently, silymarin/silybin and their preparations are advocated for the treatment of cirrhosis, chronic hepatitis and liver diseases associated with alcohol consumption and environmental toxin exposure. Known properties of silymarin/silybin are antioxidant activity in various models, inhibition of AMP-dependant phosphodiesterase, antihepatotoxic activity, anti-inflammatory activity. Silybin can be purified from milk thistle extract to a minimum purity of 90%. Silybin can be used at doses between 0.0001% and 5%, preferentially at doses between 0.001 % and 0.5% and more preferentially at doses between 0.02% and 0.2%.

In one embodiment of the invention the compositions further comprise at least one agent selected from the group consisting of isosilybin, dehydrosilybin, silychristin, silydianin, and taxifolin.

### Arctiin /Artigenin

Arctiin and artigenin are lignans isolated from the seeds and leaves of Arctium lappa (burdock). Arctiin (CAS n° 20362-31-6) is the 4-glucoside of artigenin and has been also isolated from fruit and flowers of *Forsythia viridissima* L and aerial parts of *Saussurea medusa* as shown in the following formula: 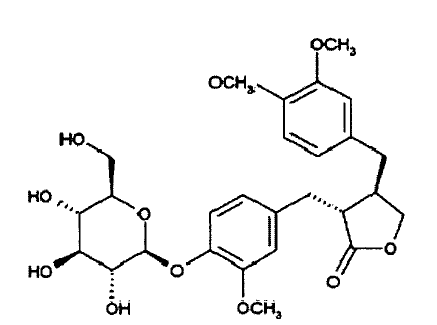 Arctiin and artigenin have been reported to show a variety of biological activities such as for example antagonist effect on the PAF receptor (Platelet-Activating Factor), anti-proliferative activity in vitro against cultured human tumor cells and anti-tumor-promoting activity. Arctiin also shows phytoestrogenic activity.

### Rhapontin

Rhapontin (synonym rhaponticin, 3,3',5-Trihydroxy-4'-methoxystilbene 3-b-D-glucopyranoside, CAS number 155-58-8) is an hydroxystilbene derivative found notably in different Rheum species according to the following formula: 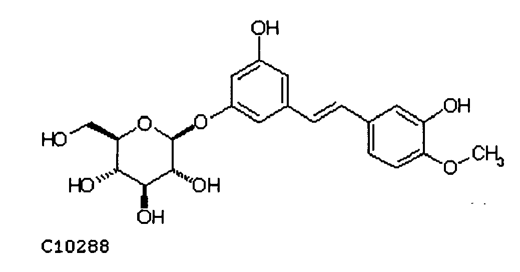 Rhapontin is said to have weak oestrogenic activity.

### Nortrachelogenin

Nortrachelogenin (CAS n° 34444-37-6, syn 8'-(R)-4,4',8-trihydroxy-3,3'-dimethoxylignanolide, dihydro-3-hydroxy-3,4-bis((4-hydroxy-3-methoxyphenyl)methyl)-2(3H) -furanone) is a lignan present in different plant species notably *Wikstroemia indica,* arnica, *Taxus cuspidate,* heartwood and knots of *Pinus silvestris* according to the following formula:

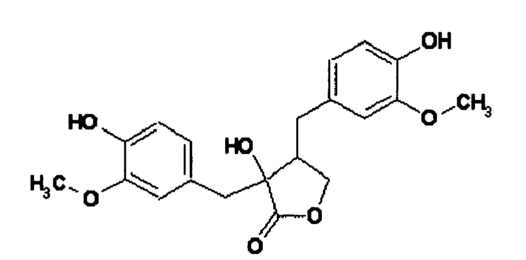

This compound shows different biological activities, like e.g. effects on the central nervous system producing depression in rabbits or for fighting tumours.

A further embodiment of the invention is directed to compositions comprising nortrachelogenine and at least one other agent selected from the group consisting of silybin, piperine, tetrahydropiperine, rhapontin, arctiin, artigenin and mixtures thereof.

### Microcapsules

The compositions according to the present invention may be applied orally or topically. In a preferred embodiment, however, either the total composition or the agent itself is encapsulated, preferably micro-encapsulated. This embodiment encompasses the encapsulation of each agent alone as well as the encapsulation of two or more or all agents present in the composition.

"Microcapsules" are understood to be spherical aggregates with a diameter of about 0.1 to about 5 mm which contain at least one solid or liquid core surrounded by at least one continuous membrane. More precisely, they are finely dispersed liquid or solid phases coated with film-forming polymers, in the production of which the polymers are deposited onto the material to be encapsulated after emulsification and coacervation or interfacial polymerization. In another process, liquid active principles are absorbed in a matrix ("microsponge") and, as microparticles, may be additionally coated with film-forming polymers. The microscopically small capsules, also known as nanocapsules, can be dried in the same way as powders. Besides single-core microcapsules, there are also multiple-core aggregates, also known as microspheres, which contain two or more cores distributed in the continuous membrane material. In addition, single-core or multiple-core microcapsules may be surrounded by an additional second, third etc. membrane. The membrane may consist of natural, semisynthetic or synthetic materials. Natural membrane materials are, for example, gum arabic, agar agar, agarose, maltodextrins, alginic acid and salts thereof, for example sodium or calcium alginate, fats and fatty acids, cetyl alcohol, collagen, chitosan, lecithins, gelatin, albumin, shellac, polysaccharides, such as starch or dextran, polypeptides, protein hydrolyzates, sucrose and waxes. Semisynthetic membrane materials are inter alia chemically modified celluloses, more particularly cellulose esters and ethers, for example cellulose acetate, ethyl cellulose, hydroxypropyl cellulose, hydroxypropyl methyl cellulose and carboxymethyl cellulose, and starch derivatives, more particularly starch ethers and esters. Synthetic membrane materials are, for example, polymers, such as polyacrylates, polyamides, polyvinyl alcohol or polyvinyl pyrrolidone.

Examples of known microcapsules are the following commercial products (the membrane material is shown in brackets) Hallcrest Microcapsules (gelatin, gum arabic), Coletica Thalaspheres (maritime collagen), Lipotec Millicapseln (alginic acid, agar agar), Induchem Unispheres (lactose, microcrystalline cellulose, hydroxypropylmethyl cellulose), Unicetin C30 (lactose, microcrystalline cellulose, hydroxypropylmethyl cellulose), Kobo Glycospheres (modified starch, fatty acid esters, phospholipids), Softspheres (modified agar agar) and Kuhs Probiol Nanospheres (phospholipids).

The agents are released from the microcapsules by mechanical, thermal, chemical or enzymatic destruction of the membrane, normally during the use of the preparations containing the microcapsules. Despite the fact that the state of the art a huge range of possibilities for the encapsulation of actives (= agents), methods according to which a shell is obtained by coazervation, precipitation or polycondensation of anionic and cationic polymers has been quite suitable for the formation of stable capsules. Particularly, a preferred process for the encapsulation of agents according to the present invention is characterised in that it comprises the steps of
(a) preparing a matrix from gel formers, cationic polymers and agents;
(b) optionally dispersing said matrix in an oil phase; and
(c) treating said dispersed matrix with aqueous solutions of anionic polymers and optionally removing the in phase in the process.

Of course, anionic and cationic polymers in steps (a) and (c) can be exchanged.

### • Gel formers

In the context of the invention, preferred gel formers are substances which are capable of forming gels in aqueous solution at temperatures above 40° C. Typical examples of such gel formers are heteropolysaccharides and proteins. Preferred thermogelling heteropolysaccharides are agaroses which may be present in the form of the agar agar obtainable from red algae, even together with up to 30% by weight of non-gel-forming agaropectins. The principal constituent of agaroses are linear polysaccharides of Galactose and 3,6-anhydro-L-galactose with alternate 1,3- and 1,4-glycosidic bonds. The heteropolysaccharides preferably have a molecular weight of 110,000 to 160,000 and are both odourless and tasteless. Suitable alternatives are pectins, xanthans (including xanthan gum) and mixtures thereof. Other preferred types are those which in 1 % by weight aqueous solution still form gels that do not melt below 80° C. and solidify again above 40° C. Examples from the group of thermogelling proteins are the various gelatines.

### • Anionic polymers

Salts of alginic acid are preferred for this purpose. The alginic acid is a mixture of carboxyl-containing polysaccharides with the following idealized monomer unit:

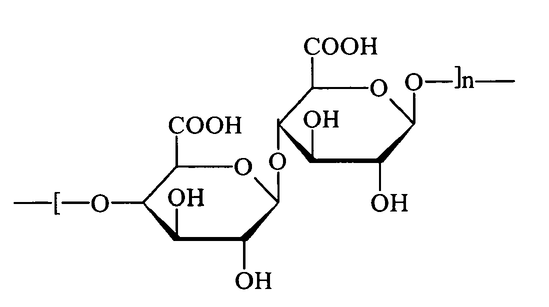

The average molecular weight of the alginic acid or the alginates is in the range from 150,000 to 250,000. Salts of alginic acid and complete and partial neutralization products thereof are understood In particular to be the alkali metal salts, preferably sodium alginate ("algin") and the ammonium and alkaline earth metal salts. Mixed alginates, for example sodium/magnesium or sodium/calcium alginates, are particularly preferred. In an alternative embodiment of the invention, however, carboxymethyl celluloses and anionic chitosan derivatives, for example the carboxylation and above all succinylation products are also suitable for this purpose.

### • Cationic polymers

Chitosans are biopolymers which belong to the group of hydrocolloids. Chemically, they are partly de-acetylated chitins differing in their molecular weights which contain the following - idealized - monomer unit:

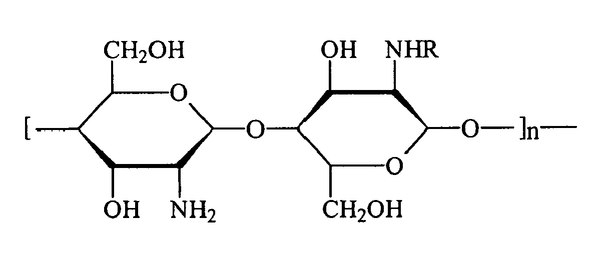

In contrast to most hydrocolloids, which are negatively charged at biological pH values, chitosans are cationic biopolymers under these conditions. The positively charged chitosans are capable of interacting with oppositely charged surfaces and are therefore used in cosmetic hair- care and body-care products and pharmaceutical preparations. Chitosans are produced from chitin, preferably from the shell residues of crustaceans which are available in large quantities as inexpensive raw materials. In a common process the chitin is normally first de-proteinized by addition of bases, de-mineralized by addition of mineral acids and, finally, de-acetylated by addition of strong bases, the molecular weights being distributed over a broad spectrum. Preferred types are those which are disclosed in German patent applications DE 4442987 A1 and DE 19537001 A1 (Henkel) and which have an average molecular weight of 10,000 to 500,000 Dalton or 800,000 to 1,200,000 Dalton and/or a Brookfield viscosity (1% by weight in glycolic acid) below 5,000 mPas, a degree of de-acetylation of 80 to 88 % and an ash content of less than 0.3% by weight. In the interests of better solubility in water, the chitosans are generally used in the form of their salts, preferably as glycolates.

In a preferred embodiment of the invention a 1 to 10 and preferably 2 to 5% by weight aqueous solution of the gel former, preferably agar agar, is normally prepared and heated under reflux. A second aqueous solution containing the cationic polymer, preferably chitosan, in quantities of 0.1 to 2 and preferably 0.25 to 0.5% by weight and the active principle (= agent) in quantities of 0.1 to 25 and preferably 0.25 to 10% by weight is added in the boiling heat, preferably at 80 to 100° C.; this mixture is called the matrix. Accordingly, the charging of the microcapsules with active principles may also comprise 0.1 to 25% by weight, based on the weight of the capsules. If desired, water-insoluble constituents, for example inorganic pigments, may also be added at this stage to adjust viscosity, generally in the form of aqueous or aqueous/alcoholic dispersions. In addition, to emulsify or disperse the active principles, it can be useful to add emulsifiers and/or solubilisers to the matrix. After its preparation from gel former, cationic polymer and active principle, the matrix optionally is very finely dispersed in an oil phase with intensive shearing in order to produce small particles in the subsequent encapsulation process. It has proved to be particularly advantageous in this regard to heat the matrix to temperatures in the range from 40 to 60°C while the oil phase is cooled to 10 to 20°C. The actual encapsulation, i.e. formation of the membrane by contacting the cationic polymer in the matrix with the anionic polymers, takes place in the third step. To this end, it is advisable to wash the matrix - dispersed in the oil phase - with an aqueous ca. 0.1 to 3 and preferably 0.25 to 0.5% by weight aqueous solution of the anionic polymer, preferably the alginate, at a temperature in the range from 40 to 100 and preferably 50 to 60° C. and, at the same time, to remove the oil phase if present. The resulting aqueous preparations generally have a microcapsule content of 1 to 10% by weight. In some cases, it can be of advantage for the solution of the polymers to contain other ingredients, for example emulsifiers or preservatives. After filtration, microcapsules with a mean diameter of preferably 1 to 3 mm are obtained. It is advisable to sieve the capsules to ensure a uniform size distribution. The microcapsules thus obtained may have any shape within production-related limits, but are preferably substantially spherical.

### Industrial application

One embodiment of the invention is directed to the use of silybin in combination with piperine and/or tetrahydropiperine for the manufacture of/ in a cosmetic composition.

It has surprisingly been found that the cosmetic compositions comprising silybin in combination with piperine and/or tetrahydropiperine are suitable for the prevention and/or soothing of dry skin. A further embodiment of the invention is therefore directed to the use of silybin in combination with piperine and/or tetrahydropiperine for the prevention and/or soothing of dry skin and/or for the prevention and/or soothing of reactive skin against external stresses.

A further embodiment of the invention is directed to the use of an agent selected from the group consisting of silybin, piperine, tetrahydropiperine, nortrachelogenine, rhapontin, arctiin and artigenin in a cosmetic composition for the prevention and/or soothing of dry skin or reactive skin against external stresses.

Such external stresses are for example UVB radiation, chemicals, pollution, temperature variation, inappropriate cosmetic products.

### Cosmetic compositions

The compositions according to the present invention may contain in addition surfactants, oil bodies, emulsifiers, superfatting agents, pearlising waxes, consistency factors, polymers, silicone compounds, waxes, stabilizers, primary and secondary sun protection agents, biogenic agents, hydrotropes, preservatives, solubilizers, perfume oils, dyes and the like as additional auxiliaries and additives.

In a further embodiment of the invention the cosmetic compositions comprise at least one further substance selected from the group consisting of surfactants, oil bodies, emulsifiers, superfatting agents, pearlising waxes, consistency factors, polymers, silicone compounds, waxes, stabilizers, primary and/or secondary sun protection agents, biogenic agents, hydrotropes, preservatives, solubilizers, perfume oils, dyes and mixtures thereof.

### Surfactants

Preferred surfactants are anionic and/or amphoteric or zwitterionic surfactants. Typical examples of anionic surfactants are soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligoglucoside sulfates, protein fatty acid condensates (particularly wheat-based vegetable products) and alkyl (ether) phosphates. If the anionic surfactants contain polyglycol ether chains, they may have a conventional homolog distribution although they preferably have a narrow-range homolog distribution. Typical examples of amphoteric or zwitterionic surfactants are alkylbetaines, alkylamidobetaines, aminopropionates, aminoglycinates, imidazolinium betaines and sulfobetaines. The surfactants mentioned are all known compounds. Information on their structure and production can be found in relevant synoptic works, cf. for example J. Falbe (ed.), "Surfactants in Consumer Products", Springer Verlag, Berlin, 1987, pages 54 to 124 or J. Falbe (ed.), "Katalysatoren, Tenside und Mineralöladditive (Catalysts, Surfactants and Mineral Oil Additives)", Thieme Verlag, Stuttgart, 1978, pages 123-217**.** The percentage content of surfactants in the preparations may be from 0.1 to 10% by weight and is preferably from 0.5 to 5% by weight, based on the preparation.

### Oil bodies

In a preferred embodiment of the invention the excipient is an oil component (= oil body). Suitable oil bodies, which can form constituents of the O/W emulsions, are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C ₁₃-carboxylic acids with linear or branched C₆-C ₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆ -C₁₀-fatty acids, liquid mono-/di-/triglyceride mixtures based on C₆-C₁₈-fatty acids, esters of C₆- C₂₂-fatty alcohols and/or Guerbet alcohols with aromatic carboxylic acids, in particular benzoic acid, esters of C₂- C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol® CC), Guerbet carbonates, based on fatty alcohols having 6 to 18, preferably 8 to 10, carbon atoms, esters of benzoic acid with linear and/or branched C₆-C₂₂-alcohols (e.g. Finsolv® TN), linear or branched, symmetrical or asymmetrical dialkyl ethers having 6 to 22 carbon atoms per alkyl group, such as, for example, dicaprylyl ether (Cetiol® OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.

### Emulsifiers

The cosmetic compositions can further comprise emulsifiers, including for example:
• products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
• C_{12/18} fatty acid monoesters and diesters of addition products of 1 to 30 mol ethylene oxide onto glycerol;
• glycerol mono- and diesters and sorbitan mono- and diesters of saturated and unsaturated fatty acids containing 6 to 22 carbon atoms and ethylene oxide addition products thereof;
• addition products of 15 to 60 mol ethylene oxide onto castor oil and/or hydrogenated castor oil;
• polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
• addition products of 2 to 15 mol ethylene oxide onto castor oil and/or hydrogenated castor oil;
• partial esters based on linear, branched, unsaturated or saturated C_{6/22} fatty acids, ricinoleic acid and 12-hydroxystearic acid and glycerol, polyglycerol, pentaerythritol, - dipentaerythritol, sugar alcohols (for example sorbitol), alkyl glucosides (for example methyl glucoside, butyl glucoside, lauryl glucoside) and polyglucosides (for example cellulose);
• mono-, di and trialkyl phosphates and mono-, di- and/or tri-PEG-alkyl phosphates and salts thereof;
• wool wax alcohols;
• polysiloxane/polyalkyl polyether copolymers and corresponding derivatives;
• mixed esters of pentaerythritol, fatty acids, citric acid and fatty alcohol and/or mixed esters of C₆₋₂₂ fatty acids, methyl glucose and polyols, preferably glycerol or polyglycerol,
• polyalkylene glycols and
• glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:

### • Partial glycerides

Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

### • Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

### • Polyglycerol esters

Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

Typical anionic emulsifiers are aliphatic C₁₂₋₂₂ fatty acids, such as palmitic acid, stearic acid or behenic acid for example, and C₁₂₋₂₂ dicarboxylic acids, such as azelaic acid or sebacic acid for example.

Other suitable emulsifiers are zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### Superfatting agents

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

### Consistency factors

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents

Suitable thickeners are polymeric thickeners, such as Aerosil® types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400®, cationic starch, copolymers of diallyl ammonium salts and acrylamides, quaternized vinyl pyrrolidone/vinyl imidazole polymers such as, for example, Luviquat® (BASF), condensation products of polyglycols and amines, quaternized collagen polypeptides such as, for example, Lauryldimonium Hydroxypropyl Hydrolyzed Collagen (Lamequat® L, Grunau), quaternized wheat polypeptides, polyethyleneimine, cationic silicone polymers such as, for example, amodimethicone, copolymers of adipic acid and dimethylaminohydroxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®}A-15, Mirapol^{®} AD-1, Mirapol^{®} AZ-1 of Miranol.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Pearlising waxes

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy-substituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91,27 (1976**).**

### Waxes

Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

### Stabilizers

Metal salts of fatty acids such as, for example, magnesium, aluminium and/or zinc stearate or ricinoleate may be used as stabilizers.

### Primary sun protection factors

Primary sun protection factors in the context of the invention are, for example, organic substances (light filters) which are liquid or crystalline at room temperature and which are capable of absorbing ultraviolet radiation and of releasing the energy absorbed in the form of longer-wave radiation, for example heat. UV-B filters can be oil-soluble or water-soluble. The following are examples of oil-soluble substances:
• 3-benzylidene camphor or 3-benzylidene norcamphor and derivatives thereof, for example 3-(4-methylbenzylidene)-camphor;
• 4-aminobenzoic acid derivatives, preferably 4-(dimethylamino)benzoic acid-2-ethylhexyl ester, 4-(dimethylamino)-benzoic acid-2-octyl ester and 4-(dimethylamino)benzoic acid amyl ester;
• esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene);
• esters of salicylic acid, preferably salicylic acid-2-ethylhexyl ester, salicylic acid-4-isopropylbenzyl ester, salicylic acid homomenthyl ester;
• derivatives of benzophenone, preferably 2-hydroxy-4-methoxybenzophenone, 2-hydroxy-4-methoxy-4'-methylbenzophenone, 2,2'-dihydroxy-4-methoxybenzophenone;
• esters of benzalmalonic acid, preferably 4-methoxybenzalmalonic acid di-2-ethylhexyl ester;
• triazine derivatives such as, for example, 2,4,6-trianilino-(p-carbo-2-ethyl-1'-hexyloxy)-1,3,5-triazine and Octyl Triazone or Dioctyl Butamido Triazone (Uvasorb^{®} HEB);
• propane-1,3-diones such as, for example, 1-(4-tert.butylphenyl)-3-(4'-methoxyphenyl)-propane-1,3-dione;
• ketotricyclo(5.2.1.0)decane derivatives.

Suitable water-soluble substances are
• 2-phenylbenzimidazole-5-sulfonic acid and alkali metal, alkaline earth metal, ammonium, alkylammonium, alkanolammonium and glucammonium salts thereof;
• sulfonic acid derivatives of benzophenones, preferably 2-hydroxy-4-methoxybenzophenone-5-sulfonic acid and salts thereof;
• sulfonic acid derivatives of 3-benzylidene camphor such as, for example, 4-(2-oxo-3-bornylidenemethyl)-benzene sulfonic acid and 2-methyl-5-(2-oxo-3-bomylidene)-sulfonic acid and salts thereof.

Typical UV-A filters are, in particular, derivatives of benzoyl methane such as, for example, 1-(4'-tert.butylphenyl)-3-(4'-methoxyphenyl)-propane-1,3-dione, 4-tert.butyl-4'-methoxydibenzoyl methane (Parsol^{®} 1789) or 1-phenyl-3-(4'-isopropylphenyl)-propane-1,3-dione and the enamine compounds (BASF). The UV-A and UV-B filters may of course also be used in the form of mixtures. Particularly favourable combinations consist of the derivatives of benzoyl methane, for example 4-tert.butyl-4'-methoxydibenzoyl methane (Parsol^{®} 1789) and 2-cyano-3,3-phenylcinnamic acid-2-ethylhexyl ester (Octocrylene^{®}), in combination with esters of cinnamic acid, preferably 4-methoxycinnamic acid-2-ethylhexyl ester and/or 4-methoxycinnamic acid propyl ester and/or 4-methoxycinnamic acid isoamyl ester. Combinations such as these are advantageously combined with water-soluble filters such as, for example, 2-phenylbenzimidazole-5-sulfonic acid and alkali metal, alkaline earth metal, ammonium, alkylammonium, alkanolammonium and glucammonium salts thereof.

### Secondary sun protection factors

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propyl-thiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

### Biogenic agents

In the context of the invention, biogenic agents are, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, for example prune extract, bambara nut extract, and vitamin complexes.

### Enzyme inhibitors

Suitable enzyme inhibitors are, for example, esterase inhibitors. These are preferably trialkyl citrates, such as trimethyl citrate, tripropyl citrate, triisopropyl citrate, tributyl citrate and, in particular, triethyl citrate (Hydagen CAT). The substances inhibit enzyme activity, thereby reducing the formation of odour. Other substances which are suitable esterase inhibitors are sterol sulfates or phosphates, such as, for example, lanosterol, cholesterol, campesterol, stigmasterol and sitosterol sulfate or phosphate, dicarboxylic acids and esters thereof, such as, for example, glutaric acid, monoethyl glutarate, diethyl glutarate, adipic acid, monoethyl adipate, diethyl adipate, malonic acid and diethyl malonate, hydroxycarboxylic acids and esters thereof, such as, for example, citric acid, malic acid, tartaric acid or diethyl tartrate, and zinc glycinate.

### Hydrotropes

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
• glycerol;
• alkylene glycols such as, for example, ethylene glycol, diethylene glycol, propylene glycol, butylene glycol, hexylene glycol and polyethylene glycols with an average molecular weight of 100 to 1000 Dalton;
• technical oligoglycerol mixtures with a degree of self-condensation of 1.5 to 10, such as for example technical diglycerol mixtures with a diglycerol content of 40 to 50% by weight;
• methylol compounds such as, in particular, trimethylol ethane, trimethylol propane, trimethylol butane, pentaerythritol and dipentaerythritol;
• lower alkyl glucosides, particularly those containing 1 to 8 carbon atoms in the alkyl group, for example methyl and butyl glucoside;
• sugar alcohols containing 5 to 12 carbon atoms, for example sorbitol or mannitol,
• sugars containing 5 to 12 carbon atoms, for example glucose or sucrose;
• amino sugars, for example glucamine;
• dialcoholamines, such as diethanolamine or 2-aminopropane-1,3-diol.

### Preservatives

Suitable preservatives are, for example, phenoxyethanol, formaldehyde solution, parabens, pentanediol or sorbic acid and the other classes of compounds listed in **Appendix 6, Parts A and B of the Kosmetikverordnung ("Cosmetics Directive").**

### Perfume oils

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### Dyes

Suitable dyes are any of the substances suitable and approved for cosmetic purposes as listed, for example, in the publication **"**Kosmetische Färbemittel" of the Farbstoffkommission der Deutschen Forschungsgemeinschaft, Verlag Chemie, Weinheim, 1984, pages 81 to 106**.** Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. These dyes are normally used in concentrations of 0.001 to 0.1 % by weight, based on the mixture as a whole.

The total percentage content of auxiliaries and additives may be from 1 to 50% by weight and is preferably from 5 to 40% by weight, based on the particular composition. The compositions may be produced by standard hot or cold processes.

### Examples

### Examples: Cosmetic compositions comprising silybin and piperine

### Cream formulation 1

| Ingredient [INCI] | % by weight |
|---|---|
| Emulgade® SE-PF ⁽²⁾ | 5.00 |
| [Glyceryl Stearate (and) Ceteareth-20 (and) | |
| Ceteareth-12 (and) Ceteareth Alcohol (and) Cetyl Palmitate] | |
| Lanette®22 ⁽²⁾ [Behenyl alcohol] | 2.00 |
| Cegesoft® C24 ⁽²⁾ (Ethylhexylpalmitate) | 4.00 |
| Cetiol® PGL ⁽²⁾ [Hexyldecanol, Hexyldecyl Laurate] | 4.00 |
| Eumulgin® B2 ⁽²⁾ [Ceteareth- 20] | 0.40 |
| DC 200-350cts ⁽³⁾ [Dimethicone] | 1.00 |
| PEG7 Glyceryl cocoate | 1.00 |
| Butylene glycol | 2.00 |
| Cosmedia® SP ⁽²⁾ [Sodium Polyacrylate] | 0.80 |
| Plantapon® ACG 35 ⁽²⁾ [Disodium Cocoyl Glutamate] | 0.80 |
| Elestab® 50J ⁽¹⁾ [Chlorphensin and Methylparaben] | 0.40 |
| Glycerine | 4.00 |
| Piperin | 0.10 |
| Silybin | 0.20 |
| Deionized water | add up to 100 |

### Cream formulation 2

| Ingredient [INCI] | % by weight |
|---|---|
| Eumulgin® VL 75 ⁽²⁾ | 4,00 |
| [Lauryl Glucoside (and) Polyglyceryl-2 Dipoly- | |
| hydroxystearate (and) Glycerin] | |
| Lanette® 22 ⁽²⁾ [Behenyl alcohol] | 2.00 |
| Cetiol® 868 ⁽²⁾ [Ethylhexyl Stearate] | 4.00 |
| Cetiol® OE ⁽²⁾ [Dicaprylylether] | 2.00 |
| Cetiol® PGL ⁽²⁾ [Hexyldecanol, Hexyldecyl Laurate] | 4.00 |
| DC 200-350cts ⁽³⁾ [Dimethicone] | 1.00 |
| Cosmedia® SP ⁽²⁾ [Sodium Polyacrylate] | 1.00 |
| Elestab® 388 ⁽¹⁾ | 2.50 |
| [Propylene Glycol and Phenoxyethanol and | |
| Chlorphensin and Methylparaben] | |
| Keltrol® CGT⁽⁴⁾ [Xanthan Gum] | 0.25 |
| Glycerin | 3.00 |
| Plantapon® ACG 35 ⁽²⁾ [Disodium Cocoyl Glutamate] | 0.80 |
| Piperin | 0.50 |
| Silybin | 0.10 |
| Deionized water | add up to 100 |

### Cream formulation 3

| Ingredient [INCI] | % by weight |
|---|---|
| Emulgade® NLB⁽²⁾ | 4.00 |
| [Steareth-2 (and) Ceteareth-12 (and) Stearyl Alcohol (and) | |
| Ceteareth-20 (and) Distearyl Ether] | |
| Lanette® 22 ⁽²⁾ [Behenyl alcohol] | 1.50 |
| Cutina® PES⁽²⁾ [Pentaerythrityl Distearate] | 1.00 |
| Cegesoft® C24 ⁽²⁾ (Ethylhexylpalmitate) | 4.00 |
| Cetiol® LC⁽²⁾ [Coco caprylate/caprate] | 3.00 |
| Cetiol® CC⁽²⁾ [Dicaprylyl Carbonate] | 2.00 |
| Cosmedia® SP⁽²⁾ [Sodium Polyacrylate] | 0.70 |
| DC 200-350cts ⁽³⁾ [Dimethicone] | 1.00 |
| Glycerin | 3.00 |
| Elestab® 388 ⁽¹⁾ | 2.50 |
| [Propylene Glycol and Phenoxyethanol and | |
| Chlorphensin and Methylparaben] | |
| Eumulgin® SG ⁽²⁾ [Sodium Stearoyl Glutamate] | 1.00 |
| Silybin | 0.10 |
| Piperine | 0.50 |
| Deionized water | add up to 100 |

### Hair lotion

| Ingredient [INCI] | % by weight |
|---|---|
| Ethanol | 18.20 |
| Elestab® 50J ⁽¹⁾ [Chlorphensin and Methylparaben] | 0.30 |
| Piperin | 0.04 |
| Silybin | 0.02 |
| Citric Acid 10% | qsfpH 5.5 |
| Deionized water | add up to 100 |

### Hair balm

| Ingredient [INCI] | % by weight |
|---|---|
| Dehyquart® C 4046 ⁽²⁾ | 3.50 |
| [Cethearyl Alcohol and Dipalmitoylethyl Hydroxyethylmonium Methosuflate and Ceteareath-20] | |
| Eumulgin® B2 ⁽²⁾ [Ceteareth-20] | 0.40 |
| Lanette® O ⁽²⁾ [Cetearyl Alcohol] | 2.50 |
| Cetiol® J 600 ⁽²⁾ [Oleyl Erucate] | 3.00 |
| Butylene glycol | 0.20 |
| PEG7 Glyceryl cocoate | 0.10 |
| Elestab® 388 ⁽¹⁾ | 2.50 |
| [Propylene Glycol and Phenoxyethanol and | |
| Chlorphensin and Methylparaben] | |
| Glycerin | 2.00 |
| Cosmedia® Guar C 261⁽²⁾ | |
| [Guar Hydroxypropyltrimonium Chloride] | 0.15 |
| Piperin | 0.02 |
| Silybin | 0.02 |
| Deionized water | add up to 100 |

### Suppliers:

(1) Laboratoires Sérobiologiques. (2) Cognis, (3) Dow Corning, (4) Kelco

## Claims

1. Compositions comprising in a cosmetically acceptable excipient
(a) silybin and
(b) piperine and/or tetrahydropiperine

2. Compositions according to Claim 1, **characterised in that** the composition further comprises at least one agent selected from the group consisting of nortrachelogenine, rhapontin, arctiin, artigenin and mixtures thereof.

3. Compositions according to any of the claims 1 to 2, **characterised in that** said agents are present in amounts of 0.0001 to 5 % b.w. - calculated on the total composition.

4. Compositions according to any of the claims 1 to 3, **characterised in that** said excipient is an oil component.

5. Compositions according to any of the claims 1 to 4, **characterised in that** said compositions or said agents are encapsulated.

6. Use of silybin in combination with piperine and/or tetrahydropiperine for the manufacture of/ in a cosmetic composition.

7. Use according to claim 6 for the prevention and/or soothing of dry skin.

8. Use according to claim 6 for the prevention and/or soothing of reactive skin against external stresses.

9. Use of an agent selected from the group consisting of silybin, piperine, tetrahydropiperine, nortrachelogenine, rhapontin, arctiin and artigenin in a cosmetic composition for the prevention and/or soothing of dry skin or reactive skin against external stresses.
